# EUROPEAN PATENT SPECIFICATION

(11) **EP 3 013 159 B1**
(45) Date of publication and mention of the grant of the patent: **13.11.2019**
(21) Application number: 14735541.6
(22) Date of filing: 27.06.2014
(51) Int. Cl.: A23L 33/10, A23L 33/17, A23J 3/08, A23J 3/34, A23J 7/00, A23L 33/00

(54) **COMPOSITIONS AND NUTRITIONAL PRODUCTS WITH IMPROVED EMULSION STABILITY**
ZUSAMMENSETZUNGEN UND NAHRUNGSMITTELPRODUKTE MIT VERBESSERTER EMULSIONSSTABILITÄT
COMPOSITIONS ET PRODUITS NUTRITIONNELS PRÉSENTANT UN STABILITÉ D'ÉMULSION AMÉLIORÉE

(30) Priority: 27.06.2013 EP 13174091
(43) Date of publication of application: 04.05.2016
(73) Proprietor: Société des Produits Nestlé S.A., 1800 Vevey (CH)
(72) Inventor: BRAUN, Marcel, CH-3510 Konolfingen (CH)
(74) Representative: Stephen, Paula-Marie
(86) International application number: PCT/EP2014/063781
(87) International publication number: WO 2014/207247

(56) References cited:
- EP-A1- 2 436 389
- WO-A1-01/70047
- WO-A1-02/13620
- WO-A1-03/039264
- WO-A1-2008/006849
- US-A1- 2005 129 738

## Description

### Field of the invention

The invention relates to the field of nutritional compositions, in particular to protein compositions comprising hydrolysed protein and methods for the preparation thereof.

In embodiments the present invention relates to a method of preparing a protein composition, said method comprising enzymatic modification of milk lecithin using phospholipase. This protein composition is then included in nutritional products to increase emulsion quality and heat stability of the final nutritional products. The invention also relates to the protein composition obtainable by the method, to the use of the protein composition in preparing nutritional products, to methods of preparing nutritional products comprising the protein compositions, as well as to the nutritional product obtainable by the methods of the invention.

### Background of the invention

Nutritional products which comprise hydrolysed proteins are desired for many reasons. The hydrolysis of proteins reduces immunogenicity and is used for example in hypoallergenic nutritional products. Hypoallergenic (H.A.) means products with a reduced risk for initiation of allergic reactions. Recently, the FDA concluded that current scientific evidence is appropriate for consideration of a qualified health claim relating to a reduced risk of atopic dermatitis for feeding of hydrolyzed, 100% whey based formulas.

Further, hydrolysed proteins are more easily absorbed and thus are suitable for use in nutritional compounds for persons with malabsorption. Such compositions comprising hydrolysed protein are particularly useful in providing nutrition to malabsorbing patients with elevated protein requirements. Examples of such compositions are described in US5549905 (Enternal composition for pediatric patients) or US5661123 (Enteral composition for malabsorbing patients).

Patients challenged with metabolic stress and injury have a significant need for increased nutrients and energy. Severe injury, trauma and some disease states are associated with loss of the body's nutrient stores. Non-essential nutrients and substances that a body typically can synthesize in adequate supply may become limiting. Additionally, absorption of nutrients from the gut can become compromised even when there is no direct injury to the gastrointestinal system.

Patients suffering from a loss of nutrients require adequate nutritional support. A lack of adequate nutritional support can result in malnutrition associated complications, such as prolonged negative nitrogen balance and depletion of somatic and visceral protein levels. Thus, the goal of nutritional support is to maintain body mass, provide nitrogen and energy in adequate amounts to support healing, meet metabolic demands characterized by the degree of stress, and support immune function.

A traditional form of nutritional support is administering whole protein liquid feedings to the patient to remedy the protein deficiency. However, some patients requiring nutritional support have a compromised absorptive capacity and thus cannot tolerate whole protein liquid feedings. Many diseases or their consequences can cause malabsorption by impairment of either digestion or absorption. For instance, patients suffering from various types of inflammatory bowel diseases typically cannot tolerate whole protein feedings. As a result, semi-elemental and elemental protein diets were developed to treat such compromised patients.

However, in addition to the traditional inflammatory bowel type patients, semi-elemental and elemental protein diets are currently being used in other patient segments. Specific conditions where these diets are being used include, for example, total parenteral nutrition patients receiving early transitional feedings, acutely ill, catabolic patients with increased nitrogen needs yet requiring an elemental diet and critically ill patients not tolerating whole protein liquid tube feedings.

Still further, many patients currently being treated with elemental diets also require elevated protein levels. For instance, patients with Crohn's disease who are experiencing the massive losses of protein associated with protein-losing enteropathy require increased amount of protein. Likewise, patients suffering from diarrhea from hypoalbuminemia, chronic diarrhea with pressure ulcers, and HIV/AIDS related malabsorption and diarrhea require increased protein for adequate nutritional support.

Such malabsorbing patients with increased protein requirements need an elemental diet with elevated nitrogen levels to enhance nutrient absorption, replete protein stores, achieve nitrogen balance and promote anabolism. While a variety of elemental and semi-elemental diets are currently being used in an attempt to treat and/or provide nutritional requirements to such patients, the inventors of the present invention do not believe the needs of such patients are being adequately met.

Another application can be the treatment of stressed patients as described in WO 2007/080149.

WO02/13620A1 decsribes a method for producing a dairy product additive by subjecting a mixture of cream and a whey protein preparation to a homogenization/emulsification process. WO03/039264A1 describes treatment of an aqueous solution of intact whey protein with a phospholipase to improve foaming properties when whipped.

However, the provision of compositions which comprise both fat and hydrolysed protein in aqueous compositions is problematic. Intact proteins are good emulsifiers; however after even partial hydrolysis of proteins, their emulsifying properties are strongly reduced and it is difficult to get a relatively stable emulsion especially for liquid formulas. Thus, the stable emulsification of products, particularly those where all or some of the protein present is in the form of hydrolysed protein, is problematic.

It is known in the art to add artificial emulsifiers to nutritional products. Examples of such emulsifiers include mono and diacetyltartatic acid esters of mono/di-glycerides, citric acid esters of monoglycerides, sorbitan fatty acid esters, polyethylene sorbitan fatty acid esters, sucrose esters of fatty acids, lactic acid esters of monoglycerides, acetic acid esters of monoglycerides, polyglycerol esters of fatty acids, propan diol esters of fatty acids and sodium or calcium stearyl lactylates.

However, the use of artificial emulsifiers is perceived negatively by the consumer, and it is especially desirable to avoid them in compositions intended for infants and/or health support. Furthermore, the application of artificial emulsifiers are strongly limited by national regulatory bodies.

Addition of natural emulsifiers to products comprising hydrolysed proteins is also known. Examples of such emulsifiers include egg yolk as well as emulsifiers from soya. However, these emulsifiers add allergens into the products and into the manufacturing system which needs an additional wash-out step. Allergens in food products are therefore to be excluded, particularly in nutritional products intended for infants and/or health support.

Therefore, a need exists for a nutritional products with improved emulsion quality and stability, and which can be manufactured using a quick, reliable method.

### Summary of the invention

The present invention addresses at least the problems set out above.

Thus, the present invention relates to a method of preparing a protein composition comprising hydrolysed protein, said method comprising the steps of
a) providing an ingredient mix comprising at least one protein comprising a whey protein concentrate or a whey protein fraction, and milk lecithin in an amount of 20 to 100 mg per 100 g of the ingredient mix;
b) adding at least one phospholipase, wherein the at least one phospholipase is phospholipase A1 and/or phospholipase A2, and performing conversion of milk lecithin,
c) performing hydrolysis of said protein.

The invention relates in a further aspect to a protein composition comprising hydrolysed protein, said protein composition being obtainable or obtained according to the above method of the invention.

The invention relates in a further aspect to a method of preparing a nutritional product comprising the steps of
- providing the protein composition comprising hydrolysed protein according to the invention, and- adding one or more nutrients.

The invention relates in a further aspect to a nutritional product obtainable or obtained by the method of the invention.

The invention relates in a further aspect to the use of the protein composition according to the invention in the preparation of a nutritional product.

### Brief description of the figures

Figure 1 shows a comparison of infant formulas prepared by different methods. A= Whey protein concentrate (WPC) hydrolysed using trypsin preparation with relatively low indigenous phospholipase activity; B= WPC hydrolysed using same trypsin as A + phospholipase treatment (pancreatic phospholipase A2); C= WPC hydrolysed using trypsin preparation with relatively low indigenous phospholipase activity + addition of other stabilisers; D= WPC hydrolysed using trypsin with relatively high indigenous phospholipase activity.
   The bold black bars delineate the creaming up zone. The dotted black bar delineates the weak creaming up zone present in sample B. White bars are included to hide the identifying labels on the bottles.
Figure 2 shows DIC microscopy of samples shown in figure 1. A- D correspond to the samples in Figure 1.
Figure 3 shows the percent degree of homogenization for the samples A- D.
Figure 4 shows the amount of Lyso-Lecithin after phospholipase treatment in the samples A, B and D.

### Definitions

Prior to discussing the present invention in further details, the following terms and conventions will first be defined.

In the context of the present invention, mentioned percentages are weight/weight percentages unless otherwise stated.

The term "and/or" used in the context of the "X and/or Y" should be interpreted as "X", or "Y", or "X and Y".

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 1 to 8, from 3 to 7, from 4 to 9, from 3.6 to 4.6, from 3.5 to 9.9, and so forth.

All references to singular characteristics or limitations of the present invention shall include the corresponding plural characteristic or limitation, and vice versa, unless otherwise specified or clearly implied to the contrary by the context in which the reference is made.

Unless defined otherwise, all technical and scientific terms used herein have the same meaning as commonly understood by one of ordinary skill in the art.

The term "fat" refers to one or more triglycerides independent of their melting temperature. The term "fat" comprises both triglycerides that are in liquid form at 25°C, as well as triglycerides that are in solid or semi-solid form at 25°C.

The term "emulsion" is defined herein as a mixture of two or more liquids in which one is present as droplets, of microscopic or ultramicroscopic size, distributed throughout the other. Emulsions are formed provided that the liquids that are mixed have no (or a very limited) mutual solubility.

An "oil-in-water emulsion" refers to an emulsion in which the droplets are of oil or fat distributed throughout a water, or aqueous liquid.

The term "creaming" or "creaming up" refers to the phenomenon where bigger fat particles or fat particle aggregates are present and leads to a fat emulsion phase concentrating on the top of liquid products.

The term "stable emulsion" refers to emulsions in which the fat emulsion phase separation is strongly retarded. This quality criterion is related to avoiding visual fat separation and to providing a homogeneous formula containing full nutritional quality and digestibility.

The term "degree of hydrolysis" means the percentage of nitrogen in the form of free alpha-amino nitrogen as compared to total nitrogen. It is a measure of the extent to which the protein has been hydrolysed. (J Agric Food Chem 1979 27(6), pp 1256-1262)

The term "degree of homogenization" is measured by comparison of fat concentration in the compositions before and after centrifugation (1600 x g, for 15 min, middle part of sample is taken), expressed in percent. This method is used to predict the separation of fat emulsion phase during product storage (called "creaming up").

The term "hypoallergenic" (HA) refers to products with a reduced risk for initiation of allergic reactions.

The term "phospholipase" refers to an enzyme that hydrolyzes phospholipids into fatty acids and other lipophilic substances. The terms "lecitase" and "lecitinase" are synonyms, and are used interchangeably with phospholipase.

### Detailed description of the invention

The present invention relates to a method of preparing a protein composition, involving enzymatic conversion of milk lecithin using at least one phospholipase A1 and/or phospholipase A2. This protein composition is then included in nutritional products to increase emulsion quality and heat stability of the final nutritional products.

The inventors have surprisingly found that the treatment of milk lecithin with phospholipase leads to improved emulsions, even in compositions comprising hydrolysed protein. Without wishing to be bound by theory, it is believed that phospholipase treatment leads to the conversion of milk lecithin to compounds such as free fatty acids and/or lysolecithin, which acts to stabilize the emulsion.

Surprisingly, already very low concentrations of converted milk lecithin and applied phospholipase resulted in significant improvement in emulsion quality and stability.

Thus in one aspect, the present invention relates to a method of preparing a protein composition comprising hydrolysed protein, said method comprising the steps of
a) providing an ingredient mix comprising at least one protein comprising a whey protein concentrate or a whey protein fraction, and milk lecithin in an amount of 20 to 100 mg per 100 g of the ingredient mix;
b) adding at least one phospholipase, wherein the at least one phospholipase is phospholipase A1 and/or phospholipase A2, and performing conversion of milk lecithin, and c) performing hydrolysis of said protein.

The present invention in a further aspect relates to the protein composition obtainable or obtained according to the method.

### Protein hydrolysis

Protein hydrolysis may be performed prior to, simultaneously with, or subsequent to the conversion of milk lecithin. In one embodiment protein hydrolysis may be performed at at least one stage selected from the group consisting of before, simultaneously with, or subsequent to the addition of at least one phospholipase. In some embodiments, more than one protein hydrolysis step is performed. In one preferred embodiment of the method of the invention, the phospholipase step is performed simultaneously with a protein hydrolysis step.

Preferably the hydrolysis of protein is performed enzymatically. Any proteolytic enzyme or mixture of enzymes can be applied, such as proteases derived from animal, plant or microbial origin. In a particularly preferred embodiment, trypsin, trypsin-like endopeptidases or a fraction comprising trypsin is used to hydrolyse the protein. Trypsin, trypsin-like endopeptidases or trypsin containing preparations can be derived from plants, animal pancreas or from microbial fermentations. The microbes used may for example be genetically modified microorganisms. Trypsin preparations usually contain a mixture of enzymes in different proportions such as trypsin, chymotrypsin, elastin, carboxypeptidase, amylases, lipases and phospholipase. Examples of applications of proteolytic preparations using genetically modified microorganism are described in WO2012/042013.

The trypsin or trypsin-like endopeptidase may be provided in combination with one or more other proteases.

Thus, the proteolytic preparation may comprise a trypsin-like endopeptidase and/or a chymotrypsin like endopeptidase, for example produced from any microorganism.

In one embodiment, the trypsin-like endopeptidase is from a microorganism selected from a strain of *Fusarium,* preferably *Fusarium oxysporum* and/or from a strain of *Kutzneria,* preferably *Kutzneria albida*; and the chymotrypsin-like endopeptidase is from a strain of *Nocardiopsis,* preferably *Nocardiopsis sp.* EMBL CDS CAI94179, or *Metarhizium,* preferably *Metarhizium anisopliae* and/or a strain of *Brachysporiella,* preferably *Brachysporiella gayana.*

In a further embodiment, the protein hydrolysis in the method according to the invention is performed using a mixture of a trypsin-like endopeptidase from *Fusarium oxysporum* or *Kutzneria albida,* combined with a chymotrypsin-like endopeptidase from *Nocardiopsis sp. or Metarhizium anisopliae.*

Performing the conversion of milk lecithin simultaneously with protein hydrolysis provides a streamlined process.

Further embodiments of the method of preparing a protein composition comprising hydrolysed protein include the method wherein protein hydrolysis is performed prior to the addition of at least one phospholipase, and/or wherein protein hydrolysis is performed subsequent to the addition of at least one phospholipase.

Protein hydrolysates are useful in protein compositions to be used in preparation of for example hypoallergenic nutritional products, such as hypoallergenic infant formulas. Further, protein hydrolysates are desired in nutritional products designed to support health in individuals with absorption deficiencies, for example due to conditions of the intestines which are related to e.g. insufficient function of pancreas or other protease producing glands, because hydrolysed protein is easier to digest and absorb. Also for example patients challenged with metabolic stress and injury have a significant need for increased nutrients and energy.

However, the present invention also relates to methods, compositions and nutritional products wherein all or most of the protein is intact. It is envisaged that the phospholipase treatment and conversion of milk lecithin will support and/or improve emulsion stability of such final nutritional products. Thus, in this embodiment, the method comprises the step of providing an ingredient comprising milk lecithin and providing at least one phospholipase. The composition thus formed may be used in the preparation of nutritional products with improved emulsion stability.

The protein may be from any source or combination of sources. Examples include plant protein such as one or more of soya, pea, rice and/or animal protein, such as from dairy, such as whey and/or milk. In a preferred embodiment the protein is milk protein, such as selected from one or more of casein and whey protein such as beta-lactoglobulin and alpha-lactalbumin. In a particularly preferred embodiment the protein is provided in the form of whey, which may be for example a whey protein concentrate (WPC) or a whey protein fraction (WPF). WPC can be defined as whey protein enriched product, typically prepared by using membrane filtration processes, whereas whey protein fractions are more related to chromatographically enriched products or proteins enriched by precipitations. Whey may also be provided as liquid whey, or in dried form.

One embodiment of the invention relates to a method comprising the steps of adding milk lecithin to a nutritional composition comprising a protein hydrolysate, or to a protein hydrolysate itself; and addition of at least one phospholipase, such that milk lecithin is converted and for example fatty acids and lysolecithin is released from the milk lecithin, contributing to the emulsion stability of the final nutritional products.

### Lecithin

Lecithin is a term used to collectively refer to phospholipids. Examples of phospholipids which may be present in lecithin are phosphatidylinositol (PI), phosphatidyl-L-serine (PS), phosphatidylethanolamine (PE), Phosphatidylcholine (PC) and sphingomyelin (SP). The term lecithin and phospholipids are used interchangeably herein.

Lecithin is present in plants and animals, and the composition of lecithin differs amongst them. In Table 1 below, lecithin from soybean, hen egg yolk and bovine milk are compared. As is apparent, there are differences both in which phospholipids are present in the lecithin, as well as in the relative amounts of the phospholipids which are present.

Milk lecithin is different in composition as compared to other lecithins, as it contains substantial and similar concentrations of PE, PC, PS and SP. In contrast, egg lecithin contains mainly PC, while lecithin from soya beans lacks SP and contains PI and PA in higher concentrations.

Thus, the structural composition of milk lecithin is different from the structural composition of egg lecithin and soya lecithin. For this reason, lecithins from different sources find different applications.

It follows that the free fatty acids and lysolecithins generated by phospholipase action on milk lecithin are different from those from for example soya or egg.

Thus, lecithins from different origins or the fractions thereof can provide different functionalities. The optimal concentration range varies depending of lecithin type and application.

Surprisingly, the small amount of phospholipase-treated indigenous milk lecithin results in better emulsion stability as compared to added relatively high amounts of e.g. soybean lecithin.

**Table 1.**

| | **Soybean Lecithin Liquid** | **Hen egg yolk lecithin** | **Bovine Milk** |
|---|---|---|---|
| | % | % | % |
| **PI** | 15 | 9 | 6 |
| **PE** | 31 | 19 | 34 |
| **PA** | 14 | | |
| **PS** | | | 3 |
| **LPE** | | | |
| **PC** | 33 | 66 | 25 |
| **SP** | | 3 | 24 |
| **LPC** | 7 | 3 | |
| **others** | | | 8 |
| **Total PL** | 100 | 100 | 100 |

| | | | |
|---|---|---|---|
| PI= phosphatidylinositol, PA= phosphatidic acid, PS= phosphatidyl-L-serine, PE= phosphatidylethanolamin, PC= phosphatidylcholine, LPE= lyso-phosphatidylethanolamin, LPC= Lyso-phosphatidylcholine and SP= sphingomyelin. Others = Ceramides (GluCer, LacCer) | | | |

The percentages in Table 1 are based on levels measured in final liquid formula products. The concentration for application of soybean lecithin in liquid nutritional formulas can be up to 0.5 w/w % . This represents about 210 mg phospholipids per 100g formula. WPC based formula contains normally only about 34 mg phospholipids per 100g formula.

In one embodiment of the present invention the milk lecithin may be present in an amount in the range of 5 mg to 500 mg per 100 g of the ingredient mix, such as 10 to 100mg per 100g, preferably from 20 to 100 mg per 100 g ingredient mix. Generally, the higher the degree of protein hydrolysis, the higher the needed milk lecithin amount.

**Table 2**

| Nutritional formula containing low amounts of converted milk lecithin (0.03 g LPC & LPE /100g) | Degree of homogenisation |
|---|---|
| **with** 0.2% w/w soya lecithin | <40% |
| **without** soya lecithin | 84% |

| | |
|---|---|
| LPC= Lyso-phosphatidylcholine; LPE= lyso-phosphatidylethanolamine. | |

Table 2 describes the degree of homogenisation obtained in a nutritional product according to the invention, either without the addition of soya lecithin or with the addition of soya lectithin. Surprisingly, it was found that inclusion of soya lecithin did not improve emulsion quality.

Thus, the invention in one embodiment relates to a where nutritional product according to the invention does not comprise soya lecithin. In further embodiments of the invention, the only emulsifier present is converted milk lecithin.

In the context of the present application, the term "converted milk lecithin" refers to the products of the action of phospholipase on milk lecithin, including free fatty acids, lyso-phospholipid, diglycerides, phosphatidyl-compounds, phosphoglycerolipids and the remaining part (e.g. choline).

In a preferred embodiment of the method, the milk lecithin provided in a method according to the invention is native milk lecithin. The term native milk lecithin means that the milk lecithin is naturally present in an ingredient present in the ingredient mix, for example in a whey protein concentrate, and/or dairy products for example milk, whey, butter, cheese, cream and buttermilk, or combinations thereof.

Using native milk lecithin obviates the need for adding extraneous milk lecithin; or indeed extraneous lecithins from other sources such as soy lecithins or the like. This again avoids introduction of potential allergens to the protein hydrolysate and to the final nutritional product. Furthermore, this embodiment may be implemented using the same basic factory production line as previously employed, with only small changes.

Whey protein concentrate is one ingredient which would provide native milk lecithin.
One example of such WPCs is Lacprodan® DI-87 from Aria Foods (DK). Similar products are also available from e.g. Armor (F), Davisco (USA), DMV (NL), Glanbia (IR), Kerry (IR), Hilmar (USA), Fonterra (NZ). Typical lecithin concentrations are ranging from about 0.3-2 g/100g and protein concentrations from 25-90%.

It is advantageous that the naturally present milk lecithin in whey protein concentrate can be valorized, without the need of addition of a non-dairy compound.

Thus one embodiment relates to a method according to the present invention wherein the milk lecithin is included in the whey protein concentrate, i.e. that the provision of an ingredient such as protein, for example the provision of whey, for example in the form of WPC, also provides milk lecithin.

### Phospholipase

The inventors of the present invention have surprisingly found that addition of phospholipase to a composition comprising milk lecithin will lead to the conversion of milk lecithin and release of conversion products such as free fatty acids and/or Lyso-phospholipid, and that this acts to improve emulsion quality in the final nutritional products containing them.

Free fatty acids and/or lysolecithin are generated by the action of at least one phospholipase on milk lecithin. The phospholipase according to the present invention may be one or more selected from phospholipase A2 (PLA2), phospholipase A1 (PLA1) phospholipase B (PLB), phospholipase C (PLC) and/or phospholipase D (PLD).

The phospholipases PLA1 and PLA2 liberate one fatty acid molecule from a phospholipid, PLA1 at glycerol position SN1 and PLA2 at position SN2. Phospholipase B displays both PLA1 and PLA2 activities and cleaves both SN-1 and SN-2 acyl chains. Additionally there are two other phospholipase types, in particular phospholipase C (PLC) and Phospholipase D (PLD). Phospholipase C converts lecithin into diglycerides and phosphatidyl-compounds. Phospholipase D converts lecithin into phosphoglycerolipids and the remaining part (e.g. choline). The products of the action of any one of PLA1, PLA2, PLB, PLC, PLD or combinations thereof, on milk lecithin can be used as emulsifiers according to the invention. Thus, the invention in a further aspect relates to the use of the products of the action of any one of PLA1, PLA2, PLB, PLC, PLD or combinations thereof on milk lecithin, as emulsifiers.

The at least one phospholipase added in a method according to the invention is phospholipase A1 and/or phospholipase A2.

In one preferred embodiment, the composition of the invention comprises phospholipase PLA2. PLA2 is normally preferred as the liberation of the fatty acid at SN2 position creates a more pronounced polar head group and an end standing hydrophobic part at SN1 position, thus producing a more efficient emulsifier.

**Table 3**

| **mg/100g hydrolysate** | **PE** | **PC** | **PE & PC** | **LPE** | **LPC** | **SP** |
|---|---|---|---|---|---|---|
| No Phospholipase | 88 | 76 | 164 | <4 | <4 | 77 |
| Phospholipase A1 | 19 | 21 | 40 | 37 | 34 | 80 |
| Phospholipase A2 | <4 | 18 | 18 | 45 | 41 | 84 |
| Phospholipase A1 & A2 | 13 | 13 | 26 | 5 | 12 | 78 |

| | | | | | | |
|---|---|---|---|---|---|---|
| PI= phosphatidylinositol, PA= phosphatidic acid, PS= phosphatidyl-L-serine, PE= phosphatidylethanolamin, PC= phosphatidylcholine, LPE= lyso-phosphatidylethanolamin, LPC= Lyso-phosphatidylcholine and SP= sphingomyelin. Others = Ceramides (GluCer, LacCer). The percentages in Table 3 are %w/w based on levels measured in hydrolysate product. | | | | | | |

The use of phopolipase A1 and phospholipase A2 permits the production a broad variety of lecithins, for example, lecithins, lysolecithins with fatty acids at sn-1 position, lysolecithins with fatty acids at sn-2 position and non hydrolyzed milk sphingomyelin, providing good emulsion stability.

PLA1 is available from Novozymes under the trademark Phospholipase ™Ultra. PLA2 is available commercially, for example under the trademark Maxapal® A2 from DSM Food Specialties B.V., Netherlands.

Phospholipase may be present in an amount in the range of 1 µg to 10 mg per 100 g of the protein composition comprising hydrolysed protein, such as for example 10 ug to 1 mg, such as 10 ug to 5 mg, such as 20 ug to 1 mg, such as 500 ug to 1 mg; preferably from 0.01 to 0.1 mg per 100 g protein composition.

The protein compositions may contain protein in the range of 1 - 20% with a degree of hydrolysis in the range of 8 - 45%. Lecithin is present normally in the range of 5-200mg/100g protein composition.

The protein compositions of the invention may comprise for example 10 µg - 10mg phospholipase per 10 - 100 mg lecithin present; or for example 0.1 - 100 Units per about 10-100 mg lecithin presentin the protein composition.

Typically, the phospholipase treatment may be performed at a temperature from 30 to 80°C, such as from 35 to 75°C, 40 to 65°C, 40 to 60°C, preferably at 40-60°C.

The phospholipase treatment may be performed at a pH value of 5 to 9.5, such as a pH from 5 to 9, from 5.5 to 8.5, from 5.5 to 8, or from 6 to 8.

The phospholipase treatment may be from a few minutes until several hours, such as from 3 minutes to 10 hours, for typically between 30 minutes to 6 hours.

As mentioned above, the stability of the emulsion is dependent on both the amount of protein present, and the degree of hydrolysis of the protein. Thus, decreasing amounts of protein and/or increasing levels of hydrolysis of the protein will require increased levels of phospholipid conversion in order to ensure emulsion stability in the final nutritional product. The levels of phospholipid conversion can be regulated by selection of the amount of the lecithin, the amount of phospholipase and/or reaction length and other parameters. This selection is within the skill of the art.

The ratio of phospholipids to fat is an important factor for emulsion stability.

Lecithin and fat are present normally in the range of 10-200mg lecithin/100g respective 0.03 - 6g fat/100g. This corresponds to ratios of lecithin to fat of 0.2:99.8 to 67:33.

The phospholipase treatment may be terminated by the inactivation of the phospholipase. Inactivation may be achieved by for example proteolysis of the phospholipase, heat inactivation, inhibitor addition or pH inactivation. Examples of heat inactivation include UHT treatment etc.

Depending on the type of phospholipase and the applied conditions phospholipase can be inactivated by the proteolytic activity. In the case that such conditions are preferable phospholipase treatment needs to be performed before proteolytic treatment or after inactivation of proteases.

The inventors of the present invention have thus surprisingly found that small amounts of phospholipase added to a composition comprising milk lecithin will result in an improved emulsification of the composition without addition of other emulsifying agents.

Thus, a preferred embodiment of the method of the invention comprises the steps of
- providing a protein and milk lecithin, for example by providing a whey protein concentrate comprising milk lecithin
- adding at least one phospholipase, such as phospholipase A2, and
- performing hydrolysis of said protein and simultaneous phospholipase treatment

For example WPC is reconstituted to a dry matter concentration of 5 to 30 w/w %, adjusted to pH range 7 - 7.5 at 40-55°C and treated for 1 to 6 hours with proteolytic enzymes in combination with phospholipase.

### Method of preparing a protein composition

Thus in some embodiments a protein and milk lecithin is provided by providing whey, for example liquid whey. Liquid whey can for example be provided by in principle any WPC manufacturer sufficiently close to allow transport of liquid whey, which is a microbiologically sensitive raw material. Alternatively, whey powder, such as a whey protein concentrate or whey protein fraction may be used. In such cases, the whey powder is dissolved in water. For example, the whey powder may be dissolved in water which is heated, for example to a temperature in the range of from 40- 80°C, such as from 50 to 70°C, such as from 55 to 60°C, such as about 60°C.

The pH of the solution may be adjusted to a suitable pH. The pH may be selected based on the basis of the pH requirements of the enzyme or enzymes to be used in protein hydrolysis. The pH may be adjusted for example by addition of base solution.

Preferably, protein hydrolysis is performed using trypsin. The trypsin may be for example a commercial trypsin enzyme preparation, or for example may be present in commercial microbial enzyme preparation. Trypsin may be present as one enzyme in a mixture of proteolytic enzymes.

When using trypsin, the pH may be in the range of from 7.0 to 8.0, such as from 7.4 to 7.8, such as for example 7.4, 7.6, 7.7, or 7.8.

The amount of enzyme used to perform protein hydrolysis may be selected based on the amount of protein substrate.

The selection of amount of trypsin to be used is based on experimental data. The amount of protease generally depends on process conditions such as for example temperature, pH, enzymatic activity, three-dimensional structure of proteins (unfolding) and desired degree of protein hydrolysis. The selection of the amount of trypsin or enzyme preparation needed to achieve proteolysis is within the skill of the art.

The hydrolysis may be terminated by inactivation of the protein, for example heat treatment. In one example the mixture is heated to at least 80°C, such as at least 90°C , such as for example 93°C, 94°C, 95°C, 96°C, or 97°C. The mixture may then be cooled down. The hydrolysis treatment may be for for example at least 30 mins, such as in the range of from 30 mins to 8 hrs, such as about 3, 4, 5, 6, 7 or 8 hours.

Protein hydrolysis may be performed in two stages, for example comprising a first and second hydrolysis step.

The method of the invention also involves adding one or more phospholipase and performing conversion of milk lecithin to free fatty acids and lysolecithin.

The conversion of milk lecithin requires phospholipase. The one or more phospholipase may be present in the trypsin preparation, for example as indigenous pancreatic phospholipase A2. Alternatively, it may be added separately, or it may be a combination of these.

The phospholipase may be added at any suitable time in the process, for example in the first or a second hydrolysis step. Optionally phospholipase can be added at later step in the process; however it should be added before a final step for enzyme inactivation.

Process conditions need to be defined corresponding to optimum ranges for lecitase and protease enzymes.

In some embodiments, phospholipase is added in amounts from 0,1 mg till 30 mg/ kg WPC, such as from 1 to 20 mg/kg, for example 1 to 18, 1 to 16, 1 to 14, 1 to 13, 1 to 12, 1 to 10 mg/ kg WPC; or for example 5 to 25, 5 to 24, 5 to 22, 5 to 20, 5 to 15, 5 to 12 or 5 to 10 mg/ kg WPC.

In other embodiments, phospholipase may be added in amounts corresponding to from 10000 to 1000000 U/kg WPC, such as from 50000 to 500000; such as from 50000 to 250000, for example from 50000 to 225000, 50000 to 200000, 50000 to 175000; or for example 75000 to 500000, 75000 to 300000, 75000 to 250000, 80000 to 175000, 90000 to 175000, 90000 to 150000, 90000 to 130000, 100000 to 130000, 110000 to 125000 U/kg WPC, 120000 to 125000 U/kg WPC, or for example about 120000 U/kg WPC.

### The protein composition obtainable by the above method

As mentioned above, the present invention in a further aspect relates to a protein composition comprising hydrolysed protein obtainable according to the above-described methods of the invention.

The protein composition thus obtained may comprise at least peptides, amino acids, derived from whey; products of phospholipase conversion of milk lecithin, such as lysolecithin, free fatty acids; and any remaining native phospholipids derived from milk lecithin.

This protein composition may be regarded as a starting material for a process of preparing a nutritional product.

This protein composition may be in liquid form. The protein composition may alternatively be in dry form, which may thus be reconstituted.

In one embodiment, one or more oil and/or lipids is added to a protein composition obtainable by the method, and the composition is dried. Drying of the protein composition which contains protein hydrolysate and the following reconstitution can be advantageous, if oil is added before drying. The oil-containing protein composition of the invention will result in improved spray drying behaviour, better powder reconsitution and reduced foam formation during its reconstitution.

The added oil are preferably oils which are relatively stable against lipid oxidation such as medium chain triglyceride oil (MCT) or high oleic sunflower oil.

Thus, the invention in one embodiment relates to a method according to the invention comprising a drying step, and a step wherein one or more oil and/or lipids is added prior to drying.

For example, in one embodiment the invention relates to a method of the invention further comprising the steps of:
d) adding one one or more oil and/or lipids, such as medium chain triglyceride oil (MCT) or high oleic sunflower oil, and
e) drying

### Use of the protein composition of the invention

The present invention relates in a further aspect to the use of the protein composition obtainable or obtained from a method of the invention, in the preparation of a nutritional product.

The present invention also relates to the use of the protein composition obtainable or obtained according to a method of the invention as an emulsifier and/or to stabilize emulsions, for example in nutritional products such as infant formulas or health care nutrional products.

### Method of preparing a nutritional product

In a further aspect, the present invention relates to a method of preparing a nutritional product, said method comprising the steps of
a) providing the protein composition according to the invention as described above
b) adding one or more nutrients.

The one or more nutrients may be selected from for example one or more of fats, carbohydrates, minerals, trace elements, vitamins, prebiotics and probiotics.

Water may be added separately, or together with one or more of the nutrients in the form of an aqueous solution or suspension of one or more of the nutrients.

In one embodiment the method of preparing a nutritional product further comprises a drying step. The method may also further comprise a step of reconstituting the dried nutritional product in a liquid, and mixing to form an emulsion.

The method of preparing a nutritional product may further comprise steps for the homogenisation and/or sterilization of the nutritional product.

Homogenisation may be performed either before or after addition of nutrients, or alternatively before and after addition. Homogenisation may be for example between 40 - 80°C.

The nutritional product may also be sterilised. In one example, the nutritional product is sterilised by Ultra High Temperature treatment (UHT), for example by direct steam injection or indirect heating.

In one embodiment, the nutritional product may be filled and subsequently sterilized. Sterilisation may be for example from 4 to 12 minutes. The temperature of sterilisation may be for example from 118 to 124°C.

In one embodiment, the method of preparing a nutritional product may further comprise a drying step.

The drying step may be for example by spray drying. A dry nutritional product according to the invention may be reconstituted before use. Thus, in one embodiment the method of preparing a nutritional product according to the invention comprises a step of reconstituting the dried nutritional product in a liquid, and mixing to form an emulsion.

In another embodiment, one or more oil and/or lipids may be added to a nutritional product according to the invention, and the nutritional product is dried. Drying of the nutritional product of the invention and the following reconstitution can be advantageous, if oil is added before drying. The oil-containing nutritional product will result in improved spray drying behaviour, better powder reconsitution and reduced foam formation during reconstitution of the dried nutritional product according to the invention.

The added oil can be preferably oils which are relatively stable against lipid oxidation such as medium chain triglyceride oil (MCT) or high oleic sunflower oil.

Thus, the invention in one embodiment relates to a method of making a nutritional product according to the invention, wherein said method comprises a drying step, and a step wherein one or more oil and/or lipids is added prior to drying.

For example, in one embodiment the invention relates to a method of the invention further comprising the steps of
d) adding one one or more oil and/or lipids, such as medium chain triglyceride oil (MCT) or high oleic sunflower oil, and
e) drying

### A nutritional product obtainable or obtained by the method

Further aspects of the invention relate to a nutritional product obtainable according to the invented methods for preparing a nutritional product.

Thus, in one embodiment the nutritional product according to the invention is a dry composition, such as a powder, agglomerated powder products or tablets, which may be suitable for reconstitution in a liquid.

In another embodiment the nutritional product of the invention is a liquid, such as an emulsion. Preferably the emulsion is an oil-in-water emulsion. The liquid may be a ready-to-drink formula, or be a concentrate for dilution prior to consumption.

The nutritional product according to the invention may also be characterized by that it forms stable emulsions in the liquid state.

Visual observation is most relevant for characterization of an emulsion, additionally microscopy and particle size distribution (PSD) by laser diffraction (e.g. Malvern or Horiba instruments. Volume weighted mean D[3,2] values calculated form PSD data can be used to characterize emulsion stability.

In one embodiment, the invention relates to a nutritional product of the invention characterized in that the only emulsifiers present in the final nutritional product are derived from milk lecithin.

In one embodiment, the only emulsifiers present in the final nutritional product of the invention are the fatty acids, lysolecithins, mono-di-glycerides and phosphoglycerolipids derived from native milk lecithin produced by the phospholipase treatment.

In further embodiments, the nutritional product of the invention has a homogenisation degree of at least 75%, for example over 75%, for example at least 80%, at least 85%, at least 90%, at least 91%, at least 92%, or for example about 90%.

In another embodiment the invention relates to a nutritional product according to the invention, wherein the fat/aggregates have an average particle size (volume weighted mean [4,3]) of not more than 2 µm, such as not more than 1 µm, such as no more than 0.6 µm, such as below 0.6 µm, for example about 0,5 µm, or for example from 0,1 to 0.5 µm, for example from 0.2 to 0.5 µm, 0.2 to 0.5 µm, 0.3 to 0.5 µm, 0.3 to 0.4 µm.

In another embodiment, the nutritional product according to the invention has a lecithin conversion degree of at least 20%, such as at least 24%, at least 30%, at least 35%, at least 40%, at least 50%, at least 60%, at least 70%, at least 80%.

In preferred embodiments, the nutritional product has a lecithin conversion degree of at least 50%, such as at least 60%, at least 70% or at least 80%.

Thus the invention in one embodiment relates to a nutritional product having a homogenisation degree of at least 75%, and a lecithin conversion degree of at least 20% .

In yet further embodiments, the nutritional product according to the invention has a homogenisation degree of at least 75%, an average particle size of no more than 0,6 µm, and a lecithin conversion degree of at least 20%.

The nutritional products of the present invention may have an amount of hydrolyzed protein present in the range of about 1 - 8 g / 100g liquid final nutritional product, such as about 1 -5 g/100g, 1- 4 g/100g or for example about 2- 4 g/100 g liquid final nutritional product.

The nutritional products of the present invention may have an amount of phospholipase conversion products, such as lysolecithin, of at least 5 mg/100 g product, such as at least 8, at least 10, at least 12, at least 16, at least 18, at least 20 mg lysolecithin/100 g product.

The nutritional product according to the present invention may be a formula selected from the group consisting of an infant formula, a health care nutritional formula, and a nutritional supplement formula.

Infant formulas may be formulated in different ways depending on the nutritional requirements of the stage or situation of the infant. Thus, the infant formula (IF) of the invention may be selected from the group consisting of a pre-term , a term IF, a small for gestational age IF; a large for gestational age IF, post-discharge IF, a starter IF, a follow-on formula, a growing up formula, a toddler's formula, and a human milk fortifier formula.

Nutritional products of the invention which comprise hydrolysed proteins may be suitable for use for example as hypoallergenic nutritional products, such as hypoallergenic infant formulas and/or hypoallergenic health care nutritional formulas. They are also suitable for supporting nutrition in individuals who have impaired nutrient absorption, such as those suffering from various types of inflammatory bowel diseases, Crohn's disease, patients suffering from diarrhea from hypoalbuminemia, chronic diarrhea with pressure ulcers, and HIV/AIDS related malabsorption and diarrhea require increased protein for adequate nutritional support.

A health care nutritional formula refers to a formula for clinical/enteral nutrition for malabsorbing patients. Such a formula may be for example a complete elemental diet designed for the dietary management of Gastro-Intestinal-compromised patients.

A nutritional supplement formula is meant to indicate a formula which is given in addition to other foods, in order to supplement nutrition.

The nutritional product according to the invention may be for example an enteral composition or an oral composition. In one embodiment the composition may be formulated as an enteral nutritional product to be administered via feeding tube or the like, such as an oral tube feeding formula.

The nutritional product according to the invention may for example be an hypoallergenic infant formula or an hypoallergenic health care nutritional formula.

Thus, in various embodiments, the nutritional product according to the invention may be selected from the group consisting of an infant formula, a toddler's formula, a follow-on formula, a growing-up formula, an enteral nutritional product, a health care nutritional formula, an oral tube feeding formula, a preterm infant formula, a human milk fortifier.

The term "liquid final nutritional product" refers to the nutritional compositions of the invention which are either prepared in a liquid form, or, when prepared as dry formulations, the reconstituted form.

The hydrolysed protein comprised in the nutritional products of the present invention may have a degree of hydrolysis in the range of about 5 - 20%, such as 8 - 15%, such as 8 - 10%.

In one embodiment of the nutritional product of the inventions comprise lecithin conversion products such as lysolecithins and free fatty acids, and non-hydrolyzed sphingomyelin derived from milk lecithin.

Preferably, the nutritional product is an emulsion, even more preferably an oil-in-water emulsion.

Thus, in one embodiment the nutritional product of the invention comprises at least hydrolysed protein, fat, lysolecithin and free fatty acids from milk lecithin.

The invention in a further embodiment relates to the use of a nutritional product according to the invention for prevention or treatment of atopic dermatitis. In this respect, the nutritional product of the invention may be considered a medical food.

### Combination of disclosures

It should be noted that embodiments and features described in the context of one of the aspects of the present invention also apply to the other aspects of the invention.

The methods of making the protein composition and the nutritional products according to invention, as well as the protein composition and nutritional products themselves, are herein described in different parameters, such as ingredients etc. It should be noted that embodiments and features described in the context of the composition according to the invention, may also be combined with embodiments and features described in the context of the nutritional products of the invention, unless expressly stated otherwise.

All patent and non-patent references cited in the present application, are hereby incorporated by reference in their entirety.

The invention will now be described in further details in the following non-limiting examples and figures.

### Examples

### Example 1- Nutritional Composition Manufactured by Batch Hydrolysis

### Materials

Trypsin with 1250 USP U/mg and Lecitase with 10'000 IU/mg were from Novozymes. Liquid whey, defined as partially demineralized liquid whey protein concentrate at about 30-35% dry matter or whey protein concentrate powder was from Foremost Farms, Baraboo, WI, US. Lactose monohydrate powder was from Meggle (Germany).

### Method

16 kg of whey protein concentrate powder and 11 kg lactose monohydrate powder were dissolved in 54 kg of demineralized water and brought to pH 7.4 by addition of KOH 30% w/w.

The protein content was calculated by multiplication of total nitrogen concentration with factor 6.25. Based on this calculation, 65 g of trypsin was required.

Trypsin was added to a final concentration of 0.8 g per kg protein hydrolysate.

The reconstituted protein mix was adjusted to 55°C and 13 g of trypsin was added and the pH of 7.4 was kept for at least 30 min by addition KOH 30% w/w. The prehydrolyzed mix was then heat treated at 94°C for 5 min and cooled down to about 55°C. A portion of 52g trypsin, and for variant B 195 mg lecitase, was added to start the second hydrolysis. The pH was kept at 7.4 for about 3 hours and the reaction was completed by thermal inactivation of the enzymes. Finally, 9 kg of maltodextrin, 13 kg of fat mix and 1.4 kg of minerals and vitamins were added and homogenized. The final mix was further UHT treated, homogenized, aseptically filled or sterilized to produce an aseptic liquid form of the composition which is shelf stable at ambient tempearature, or spray dried to produce a powder of the composition.

### Example 2- Comparison of test variants

Four different variants were produced using the method described in Example 1.
A. Reference variant produced by the method described in Example 1, using a trypsin preparation with relatively low indigenous phospholipase activity.
B. Variant was produced using addition of 3 µg Phospholipase 10L (pancreatic phospholipase A2 ex Novozymes, 10'000 IU/ml) per g of trypsin, equivalent to about 3 mg or 11 Units per g of trypsin, added to the second hydrolysis step.
C. Variant produced in the same manner as A, but with addition of 1.2% high amylase starch and 0.03% kappa-carrageenan as stabilisers.
D. Variant produced in the same manner as A, but using a trypsin preparation with relatively high indigenous phospholipase activity.

Variant B: 3 µg Phospholipase 10L (pancreatic phospholipase A2 ex Novozymes, 10'000 IU/ml) per g of trypsin, equivalent to about 3 mg or 30 Units per g of trypsin, was added to the second hydrolysis step. This corresponds to 27 µg or 270 mUnits per 40 mg phospholipids present in 100g final formula containing about 1.5 g protein-equivalent. Protein equivalent mean proteins, peptides and amino acids. Liquid whey means partially demineralized liquid whey protein concentrate at about 30-35% dry matter.

In contrast to the known processes it has been found that a process according to an embodiment of the invention typically results in a strong increase in lysolecithins detected by HPLC.

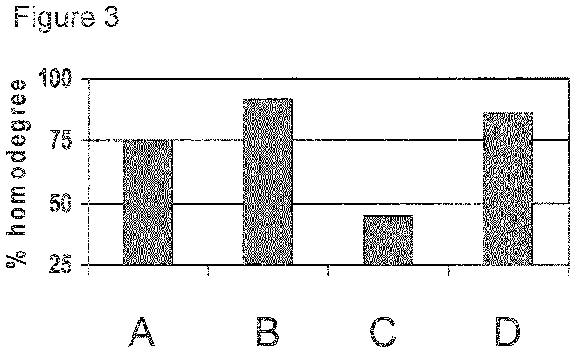

The lecithin conversion degree was increased from 24% (Variant A) to 81% (Variant B) detected by HPLC. The resulting homogenization degree was increased from 75% (Variant A) to 92% (Variant B). Degree of homogenisation is shown in Figure 3.

The variants A- D were analysed for Degree of homogenisation, Sediment value and Fat particle size volume weighted mean [4,3] and the results are shown below in Table 4.

The degree of homogenisation is measured by comparison of fat concentration in the formulas before and after centrifugation (1600 x g, for 15 min, middle part of sample is taken), expressed in percent. The higher the value, the better the emulsion quality/stability of the product. This method is used to predict the separation of fat emulsion (called "creaming") during product storage.

The sediment value represents the dried solid parts of the sample at the bottom of the centrifugation tube after determination of homogenisation degree. Generally, it is desired that this value should be low.

The Particle size volume weighted mean [4,3] is based on particle size determination by laser light diffraction. Volume based particle size equals the diameter of the sphere that has same volume as a given particle. Which equals 2*(3*volume(particle)/4/pi)^(1/3). In this product matrix it is assumed to be mainly related to fat particles/aggregates.

Generally, the lower the particle size values, the better the emulsion stability.

**Table 4**

| | Degree of homogenisation % | Sediment value % | Particle size volume weighted mean [4,3], micrometers (µm) | pH value |
|---|---|---|---|---|
| **A** | **75.4** | 0.06 | **0.515 µm** | 6.85 |
| **B** | **91.5** | 0.03 | **0.310 µm** | 6.82 |
| **C** | **44.8** | 5.43 | **3.276 µm** | 6.90 |
| **D** | **86.2** | 0.09 | **0.436 µm** | 6.88 |

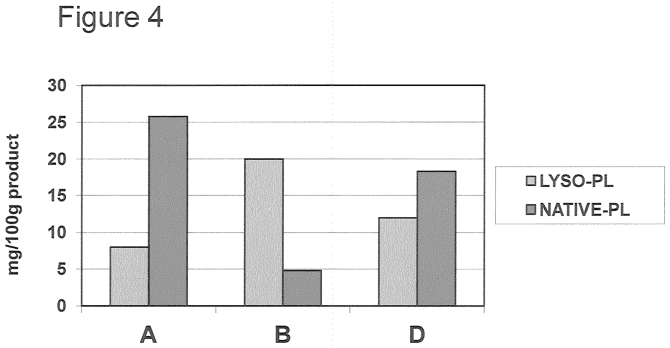

Lecithin conversion degree is shown in Figure 4 and Table 5 by presentation of non-hydrolysed lecithins vs. lyso-lecithins. Lyso-Lecithins refers to one of the products of lecithin cleavage with phospholipase A2. For good emulsion stability, the conversion of native lecithin should be high. Thus, non-hydrolysed lecithin concentrations should be low and concentration of products of lecithin conversion by phospholipase, such as lyso-lecithin, should be high.

Lecithin conversion is calculated of percentage of Lyso-L (lyso-lecithin LPE & LPC) to sum of Native-L (non hydrolyzed lecithins except SPH, sum of PI, PE, PC) and Lyso-L.

**Table 5**

| mg/100g formula | A | B | D |
|---|---|---|---|
| LYSO-L | 8 | 20 | 12 |
| Non-hydrolysed-Lecithin | 25,8 | 4,8 | 18,3 |
| Total L | 33,8 | 24,8 | 30,3 |
| Lecithin conversion degree | 24 % | 81% | 40% |

C was not analyzed as phospholipid composition of this variant is expected to be similar to that of A.

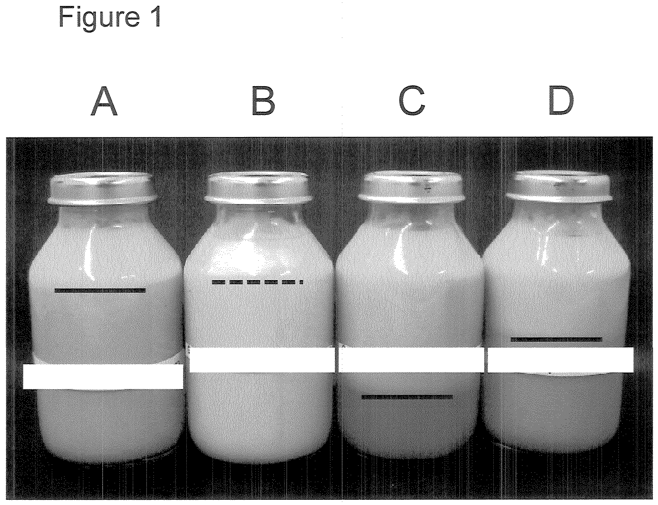

The variants A- D were further analysed and the results are shown in Figures 1, 2 and 3.

Figure 1 shows the variants A - D where strong serum formation and creaming up was shown in variants A, C, and D, whereas B shows only slight creaming.

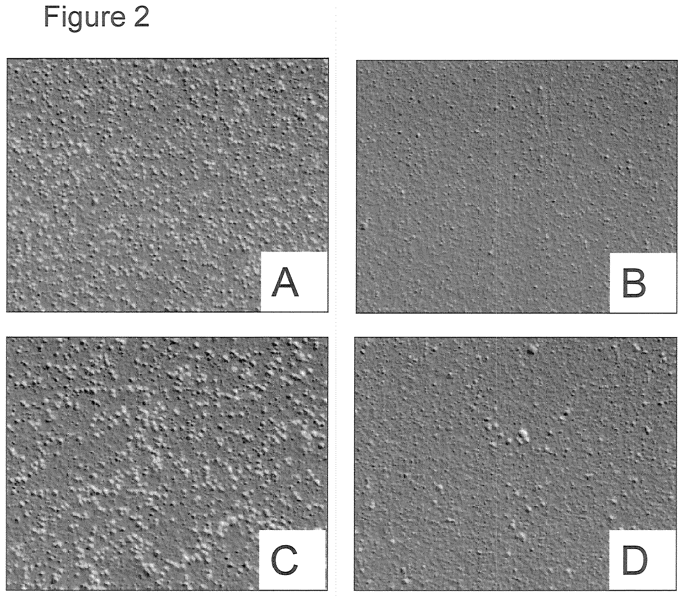

Figure 2 shows microscopy pictures of the same variants A-D, were B shows the smallest fat droplets without substantial fat droplet aggregation, followed by D with some slightly bigger fat droplets and finally A and C showing bigger fat droplets with partial fat droplet aggregation.

Figure 3 represents an overview about homogenization degree values of the products shown in Table 2. For acceptable emulsion storage stability values of at least 75% are preferred.

### Example 3- Enteral product- Nutritional Composition Manufactured by Batch Hydrolysis - Product having a moderate degree of protein hydrolysis

A nutritional composition for enteral nutrition or tube feeding for patients which cannot eat any or enough food because of an illness or may have a decreased appetite, difficulties in swallowing, or some type of surgery that interferes with eating, was produced in accordance with a method of the invention.

Liquid whey was used or whey powder was dissolved in water of 60°C to form a solution. The pH was adjusted to 7.4 for the hydrolysis reaction by addition of a base solution. The temperature of the mixture was adjusted to about 55°C and 0.6% of a commercial trypsin enzyme preparation, amount of protease based on protein substrate, was added to catalyse hydrolysis of protein in the mixture. The pH was adjusted to 7.4 for the hydrolysis reaction and maintained for at least 30 min at this pH value by addition of a base solution. After about 4 hours the hydrolysis reaction was completed and enzymes thermally inactivated. Fat was added, homogenized and the hydrolysed composition was finalized by addition of additional ingredients like minerals, trace elements, carbohydrates and vitamins. The final mix was further UHT treated, homogenised, aseptically filled, and sterilised to produce a liquid form of the composition or spray dried to produce a powder form of the composition.

The amount of phospholipase can be part of trypsin preparation as indigenous pancreatic phospholipase A2 or added before protease addition. Optionally phospholipase can be added at later steps in the process; however before a final step for enzyme inactivation.

In this example, phospholipase was part of trypsin preparation as indigenous pancreatic phospholipase A2.

**Table 6- Homogenisation degree of enteral nutrition formulas**

| Enteral nutrition formulas | Homogenisation degree |
|---|---|
| **Regular product without phospholipase** | 49% |
| **Trial product with phospholipase** | 95% |

Table 6 shows the homogenization degree of enteral nutrition formulas according to the Example 3.

### Example 4- Nutritional Composition Manufactured by Continuous Hydrolysis - Product having a medium to low degree of protein hydrolysis

A easily digestible nutritional composition was produced in accordance with a method of the invention.

Liquid whey was used or whey powder was dissolved in water of 60°C to form a solution. The pH was adjusted to 7.8 for the hydrolysis reaction by addition of a base solution. The temperature of the mixture was adjusted to about 55°C and about 1% of a commercial microbial enzyme preparation, amount of protease based on protein substrate, was continuously added to the product stream and held for about 10 minutes at about 50 to about 65°C whilst flowing through holding tubes to hydrolyse protein in the mixture. The microbial enzyme preparation was based on subtilisin, e.g. Alcalase from Novozymes. Proteolytic activity is about 2.4 AU/g compare to 6.0 AU/g for trypsin.

The enzymatic reaction was terminated by thermal inactivation of the enzyme by heat treatment. Fat was added, homogenized and the hydrolysed composition was finalized by addition of additional ingredients like minerals, trace elements, carbohydrates and vitamins. The final mix was further UHT treated, homogenised, aseptically filled, and sterilised to produce a liquid form of the composition or spray dried to produce a powder form of the composition.

The amount of phospholipase can be part of enzyme preparation or added after protease addition. Optionally phospholipase can be added at later steps in the process; however before a final step for enzyme inactivation.

### Example 5

A nutritional composition is produced as described in Example 1, with the exception that the protein hydrolysis is performed using microbial proteases.

### Example 6

A nutritional composition is produced as described in Example 3, with the exception that the protein hydrolysis is performed using a mixture of a trypsin-like endopeptidase from *Fusarium oxysporum* or *Kutzneria albida;* combined with a chymotrypsin-like endopeptidase from *Nocardiopsis sp.* or *Metarhizium anisopliae.*

### Example 7

A nutritional composition is produced as described in Example 4, with the exception that the protein hydrolysis is performed using a mixture of a trypsin-like endopeptidase from *Fusarium oxysporum* or *Kutzneria albida* combined with a chymotrypsin-like endopeptidase from *Nocardiopsis sp.* or *Metarhizium anisopliae.*

## Claims

1. A method of preparing a protein composition comprising hydrolysed protein, said method comprising the steps of
a) providing an ingredient mix comprising at least one protein comprising a whey protein concentrate or a whey protein fraction, and milk lecithin in an amount of 20 to 100 mg per 100 g of the ingredient mix;
b) adding at least one phospholipase, wherein the at least one phospholipase is phospholipase A1 and/or phospholipase A2, and performing conversion of milk lecithin,
c) performing hydrolysis of said protein.

2. The method according to claim 1 wherein protein hydrolysis is performed simultaneously with conversion of milk lecithin.

3. The method according to any of the previous claims wherein the milk lecithin is native milk lecithin.

4. The method according to any of the preceding claims further comprising the steps of
d) adding oil
e) drying

5. A protein composition comprising hydrolysed protein obtainable according to the methods according to any of claims 1 to 4.

6. A method of preparing a nutritional product, said method comprising the steps of
a) providing the protein composition according to claim 5
b) adding one or more nutrients.

7. A nutritional product obtainable by the method according to claim 6.

8. The nutritional product according to claim 7 **characterized in that** the only emulsifiers present in the nutritional product are derived from milk lecithin.

9. The nutritional product according to any of claims 7 to 8, wherein the composition is an emulsion, preferably an oil-in-water emulsion.

10. The nutritional product according to any of claims 7 to 9, wherein the composition is an enteral composition or an oral composition.

11. The nutritional product according to any of claims 7 to 10, wherein the composition is an hypoallergenic nutritional product, such as an hypoallergenic infant formula or an hypoallergenic health care nutritional formula.

12. The nutritional product according to any of claims 7 to 11 wherein the degree of lecithin conversion is at least 20%.

13. Use of the protein composition according to claim 5 in the preparation of a nutritional product.

## Patentansprüche

1. Verfahren zum Zubereiten einer Proteinzusammensetzung, umfassend hydrolysiertes Protein, das Verfahren umfassend die Schritte des
a) Bereitstellens einer Inhaltsstoffmischung, umfassend mindestens ein Protein, umfassend ein Molkenproteinkonzentrat oder eine Molkenproteinfraktion, und Milchlecithin in einer Menge von 20 bis 100 mg pro 100 g der Inhaltsstoffmischung;
b) Zusetzens von mindestens einer Phospholipase, wobei die mindestens eine Phospholipase Phospholipase A1 und/oder Phospholipase A2 ist, und Durchführens der Umwandlung von Milchlecithin,
c) Durchführens der Hydrolyse des Proteins.

2. Verfahren nach Anspruch 1, wobei die Proteinhydrolyse gleichzeitig mit der Umwandlung von Milchlecithin durchgeführt wird.

3. Verfahren nach einem der vorstehenden Ansprüche, wobei das Milchlecithin natives Milchlecithin ist.

4. Verfahren nach einem der vorstehenden Ansprüche, ferner umfassend die Schritte des
d) Zusetzens von Öl
e) Trocknens.

5. Proteinzusammensetzung, umfassend hydrolysiertes Protein, das gemäß den Verfahren nach einem der Ansprüche 1 bis 4 erhältlich ist.

6. Verfahren zum Zubereiten eines Nahrungsmittels, das Verfahren umfassend die Schritte des
a) Bereitstellens einer Proteinzusammensetzung nach Anspruch 5
b) Zusetzens eines oder mehrerer Nährstoffe.

7. Nahrungsmittel, das durch das Verfahren nach Anspruch 6 erhältlich ist.

8. Nahrungsmittel nach Anspruch 7, **dadurch gekennzeichnet, dass** die einzigen Emulgatoren, die in dem Nahrungsmittel vorliegen, von Milchlecithin abgeleitet sind.

9. Nahrungsmittel nach einem der Ansprüche 7 bis 8, wobei die Zusammensetzung eine Emulsion ist, vorzugsweise eine Öl-in-Wasser-Emulsion.

10. Nahrungsmittel nach einem der Ansprüche 7 bis 9, wobei die Zusammensetzung eine enterale Zusammensetzung oder eine orale Zusammensetzung ist.

11. Nahrungsmittel nach einem der Ansprüche 7 bis 10, wobei die Zusammensetzung ein hypoallergenes Nahrungsmittel wie eine hypoallergene Säuglingsnahrung oder eine hypoallergene Gesundheitsnährformel ist.

12. Nahrungsmittel nach einem der Ansprüche 7 bis 11, wobei der Grad der Lecithin-Umwandlung mindestens 20 % beträgt.

13. Verwendung der Proteinzusammensetzung nach Anspruch 5 bei der Zubereitung eines Nahrungsmittels.

## Revendications

1. Procédé de préparation d'une composition protéique comprenant une protéine hydrolysée, ledit procédé comprenant les étapes de
a) fourniture d'un mélange d'ingrédient comprenant au moins une protéine comprenant un concentré de protéine de lactosérum ou une fraction de protéine de lactosérum, et de la lécithine de lait en une quantité de 20 à 100 mg pour 100 g du mélange d'ingrédient ;
b) ajout d'au moins une phospholipase, dans lequel l'au moins une phospholipase est la phospholipase A1 et/ou la phospholipase A2, et conversion de la lécithine de lait,
c) hydrolyse de ladite protéine.

2. Procédé selon la revendication 1 dans lequel l'hydrolyse de la protéine est effectuée simultanément avec la conversion de la lécithine de lait.

3. Procédé selon l'une quelconque des revendications précédentes dans lequel la lécithine de lait est la lécithine de lait native.

4. Procédé selon l'une quelconque des revendications précédentes, comprenant en outre les étapes de
d) ajout d'huile
e) séchage.

5. Composition protéique comprenant une protéine hydrolysée qui peut être obtenue selon les procédés selon l'une quelconque des revendications 1 à 4.

6. Procédé de préparation d'un produit nutritionnel, ledit procédé comprenant les étapes de
a) fourniture de la composition protéique selon la revendication 5
b) ajout d'un ou plusieurs nutriments.

7. Produit nutritionnel qui peut être obtenu par le procédé selon la revendication 6.

8. Produit nutritionnel selon la revendication 7 **caractérisé en ce que** les seuls émulsifiants présents dans le produit nutritionnel sont issus de la lécithine de lait.

9. Produit nutritionnel selon l'une quelconque des revendications 7 à 8, dans lequel la composition est une émulsion, de préférence une émulsion huile-dans-l'eau.

10. Produit nutritionnel selon l'une quelconque des revendications 7 à 9, dans lequel la composition est une composition entérale ou une composition orale.

11. Produit nutritionnel selon l'une quelconque des revendications 7 à 10, dans lequel la composition est un produit nutritionnel hypoallergénique, tel qu'une formule hypoallergénique pour nourrisson ou une formule nutritionnelle hypoallergénique à visée thérapeutique.

12. Produit nutritionnel selon l'une quelconque des revendications 7 à 11, dans lequel le degré de conversion de la lécithine est d'au moins 20 %.

13. Utilisation de la composition protéique selon la revendication 5 dans la préparation d'un produit nutritionnel.
